Europäisches Patentamt

**European Patent Office**

**Office européen des brevets**

(19)

(11) Publication number: **0 087 901**
**A2**

(12)

# EUROPEAN PATENT APPLICATION

(21) Application number: 83300887.3

(22) Date of filing: 21.02.83

(51) Int. Cl.³: **A 61 M 25/00**

(30) Priority: 26.02.82 GB 8205718

(43) Date of publication of application:
07.09.83 Bulletin 83/36

(84) Designated Contracting States:
BE CH DE FR GB IT LI NL SE

(71) Applicant: AVON INDUSTRIAL POLYMERS LIMITED
Bath Road
Melksham Wiltshire SN12 8AA(GB)

(72) Inventor: Davis, Brian Edward
5 Luccombe Terrace
Bratton Wiltshire(GB)

(74) Representative: Harrison, David Christopher et al,
MEWBURN ELLIS & CO 2/3 Cursitor Street
London EC4A 1BQ(GB)

(54) **Connector for fluid communication.**

(57) A valved coupling for establishing fluid connection between two separate but couplable elements (A, B) has minimum included volume between the parts that meet together to establish that connection. This is achieved by forming the faces (4, 24) of obturators (2, 14) which meet face-to-face upon coupling to be exactly the same as each other in profile extent and boundary shape, the same being true of the marginal faces (9, 22) around the obturators which belong to valve members (1, 15) coacting with the latter, the obturators and valve members being relatively movable. Furthermore, each transition from obturator face to marginal face is smooth (collinear). Coupling means (10, 21) ensure that the elements come together with the obturators and valve members in register, respectively.

Fig.2.

EP 0 087 901 A2

## CONNECTOR FOR FLUID COMMUNICATION

This invention relates to connectors which are concerned to establish a connection for fluid communication, for example, between tubes or between a tube and a container where it is essential that contamination shall not enter into the tube.

A particular application is the connection of a drinking bottle to a tube deriving from a respirator and through which the wearer of the respirator will drink. In an extreme case the respirator may be being worn and the coupling be required when protection is needed from nuclear, biological and chemical ("NBC") conditions.

The connector proposed will however find application in other less extreme circumstances, for example in forming sterile couplings between items of medical equipment or items for chemical processing where considerable purity is required.

The difficulty is of course that standard connectors involve penetration of one element by the other in which case the penetrating element will usually have been exposed to the atmosphere and will carry contamination into the other element and/or the entrapping between the two parts of the coupling of a volume of the ambient atmosphere which is thus introduced as an impurity into the conduit formed by the connection. This is clearly intolerable where effectively total sterility or where protection against NBC conditions is required.

The present invention provides maximum security even in very extreme conditions by reducing to a line or lines the area from which external contaminants, carried on or in the vicinity of either the two elements of the coupling which are to be mated together, may be transferred into a fluid flow established through the coupling. This is achieved by having a valve member on each of the two coupling elements of the connector and an obturator normally sealing the extreme end of the duct.operable between it and the valve member, the members being relatively movable so as to permit a valving action. Each of the obturators has a face which precisely corresponds in profile, extent and boundary with the other, and the coupling elements have means for bringing those two faces into exact face-to-face registry with each other upon the mating together of the coupling elements. The margin around each obturator offered by the valve member also precisely correspond as between the two coupling elements of the connector and come into face-to-face contact upon the mating of the couplingelements. Thus upon the mating of the coupling elements an overall intimate fit is formed between the two obturators and the two marginal portions and this is interrupted only by a line or lines where the obturators seat on their respective marginal portions. There is therefore an absolutely minimal included volume between the two (this minimal included volume being provided wholly by the inevitable crack or tolerance where the extreme end of the obturator seats on the marginal portion). In a second stage of the connection the mated couplings are activated externally, to cause a relative movement of the obturators and the marginal portions whereby a duct is opened up between those parts.

To assure the mating of the coupling parts in register one or both of the valve defining parts may have a retaining element and the relative movement for opening up the duct once the coupling is connected may be assured by relative movement of the obturators and the retaining element(s).

This relative movement may be axial or may be rotational; the conenctor may establish a single or a double duct.

All the faces may be planar and coplanar; but other profiles are possible provided they correspond exactly from one element to the other.

A subsidiary aspect of the invention concerns the need for compensating for liquid withdrawn from a drinking bottle when that is being used under NBC conditions. Air cannot simply be admitted from the ambient atmosphere since it would contaminate the liquid. This is why we may, therefore, provide a double duct between the drinking bottle and the respirator.

A particular embodiment of the invention will now be described with reference to the accompanying drawings, wherein:

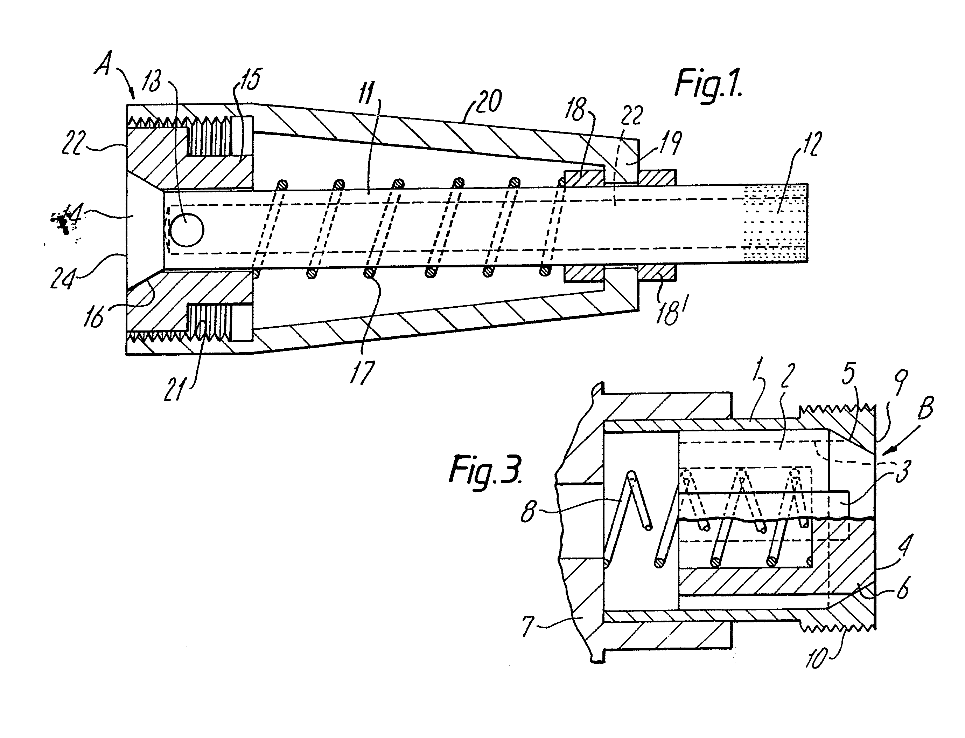

Figure 1 is a diammetrical section through the two coupling elements of a first connector before being mated together, and

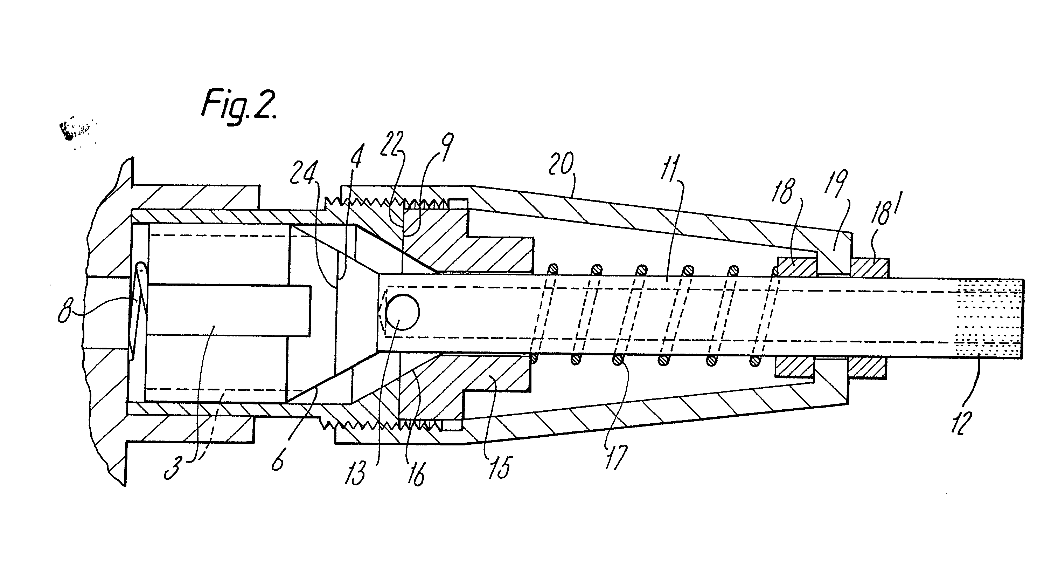

Figure 2 is the same section of the two elements mated and with the duct open.

·Looking now at the embodiment, the connector has two coupling elements generally labelled A and B.

Considering first the element B, a cylinder 1 houses a cup-like obturator 2 which is circular in transverse section. At one or more points around its diameter the cup has an axially extending inset slot 3. The mouth of the cylinder has an inwardly tapering valve-forming part 5 onto which seats a corresponding shaped portion 6 of the cup 2. These parts are very precisely formed so as to avoid any possibility of leak path between them.

The cylinder 1 is secured to or forms part of a closure 7 of a drinking bottle or to another duct- forming part with which it is desired to make a connection, a spring 8 being entrapped between that and the cup 2 to urge the cup towards the closed position where the parts 5 and 6 seat together. The extreme end face 4 of the cup is smoothly planar. The marginal portions 9 of the valve element defined by the part 5 are exactly co-planar with it when the cup is in the closed position, i.e. when the valve formed by it is seated. There is thus a single circular line of intersection between the obturator (the cup 2) and the valve member (the mouth 5) at the extreme end of the duct formed by the latter, and the face of the obturator and the face of the marginal portions of the valve are exactly collinear across each transition from one to the other. When, as here, the faces are planar, all such transitions are in a single plane.

On the outside of the cylinder 1 there is formed around its end portion retaining means 10 such as a screw thread or the slot or stud of a bayonet fitting, by which the other coupling element A can be retained in mating relationship with the element B.

Turning now to this other element A, an obturator is formed by a stem 11 which is hollow so as to offer a duct 22 which at one end opens axially, within an end portion of the stem having means 12 for retaining on it a tube to form a continuation of the duct. At the other end the duct 22 opens through a radial port 13. The stem 11 has at its extreme end beyond the port an outwardly flared part 14 which acts as obturator to a valve member in the form of a collar 15 which slides on the stem and has a flared mouth part 16 corresponding precisely to the conformation of the flared end part 14 of the stem. The collar is urged to the closed position of the valve thus formed, by a spring 17 surrounding the stem and entrapped between that collar and a bushing 18. This bushing lies within the end wall 19 of a cup-like second retaining element 20 which has on the internal periphery of its other end portion retaining means 21 for coupling with the retaining means 10. External bush 18' also surrounds the stem where it penetrates the end wall 19.

The end face 24 of the portion 14 of the stem is exactly planar as also is the end face of the marginal portion 22 of the collar 15 surrounding it and therefore the sealing effected at the extreme end of the duct formed between them is effectively at a single circular line, in that plane. This line is exactly co-extensive and registrable with the similar line between the faces 4 and 9 in the element B.

To establish the connection the two elements are mated together and retained using the retaining means 21,10. This involves an overall contraction in the length of the couplings between the end cap 7 of one element and the end wall 19 of the other. The retaining means 10,21 cause the faces 24 and 4 to meet exactly, as do also the faces of the marginal portions around them. As the contraction continues the flared surface of the part 14 becomes unseated from the corresponding surface 16 of the collar 15 and the surface 6 of the part 4 from the surface 5 thereby opening up fluid communication in the element B through the grooves 3, between the respective obturators and their seating surfaces and (in the element A) through the radial port 13. The movements of the cup 2 relative to its valve seat and of the collar 15 relative to its valve seat are not necessarily exactly equal but a sufficient degree of equality can be assured by appropriate choice of the spring rate of the springs 8,17.

It will be seen that due to the construction of the extreme ends of the ducts formed through the valve members there is substantially zero included or transferable volume when the connection is established. Even if contaminants are present on the exposed faces 4,9,22,24 of the obturators and valve members (and gross contamination can be avoided by keeping these elements in covers until they are wanted) none which are on those end faces will be transferable into the fluid stream established once the valve is opened except that truly minute amount which is entrapped exactly on the lines of parting between the obturators and their valve members.

It is to be noted that the void within the sleeve 20 which is open to the ambient atmosphere is not part of the duct which is formed through that coupling element.

This principle of line-only tranferability into the stream may also be embodied in double-duct arrangements and in those where seating or unseating is accomplished by rotational rather than axial movement.

CLAIMS:

1.      A coupling for establishing fluid connection therethrough including two separate elements (A,B) each containing an obturator (2,14) seatable on a valve seat (5,16) to act as a valve to close off a duct through that element, the valve seat being provided by a valve member (1,15) surrounding the obturator, and means (10,21) for coupling the two elements together to establish the connection characterised in that the face (4) on the obturator (2) on one element (B) corresponds exactly with a face (24) on the obturator (14) of the other element (A), a margin face (9) on the valve member (1) on one element (B) surrounding the face (4) of the obturator(2) of that element (B) corresponding exactly with a margin face (22) of the valve member (15) on the other element (A) which surrounds the face (24) of the obturator of that other element, the coupling means (10,21) being effective upon formation of the coupling to place the obturator faces (4,24) on the one hand and the margin faces (9,22) on the other in register, respectively.

2.      A coupling according to Claim 1, wherein the obturator face (4,24) and the margin face (9,22) are collinear across all transitions between them, in the seated condition of the valves.

3.    A coupling according to Claim 2, wherein the said faces (4,24,9,22) are all planar and, for each element (A or B) respectively, coplanar in the seated condition of the valves.

4.    A coupling according to Claim 1, Claim 2 or Claim 3 wherein each element has a body part (1,20), the coupling means (10,21) being on the body parts and causing upon coupling of the body parts a reduction in their overall length, coupling thereby causing unseating of the obturator (2) of the one element (B) by relative movement due to its contact with the face (4) of the obturator (14) of the other element (A), and also causing unseating of the valve member (15) of the other element (A) by virtue of contact between its marginal face (22) and the marginal face (9) of the body part (1) of the one element (A).

5.    A coupling according to Claim 4, wherein the obturator (2,15) of each element (A,B) is resiliently urged to its seated, valve-closing position, the resilient means (17) in one element (A) acting between the body part (20) and the valve member (15), the latter being mounted on a stem (11) which is a continuation of the obturator and being entrapped by the obturator.

6.      A coupling according to any one of the
preceding Claims, wherein the coupling means (10,21) are
screw-threadedly engageable together.

Fig.1.

Fig.3.

Fig.2.

2/2

0087901